**Europäisches Patentamt**

**European Patent Office**

**Office européen des brevets**

(19)

(11) Veröffentlichungsnummer: **0 289 894**
**A1**

(12) # EUROPÄISCHE PATENTANMELDUNG

(21) Anmeldenummer: **88106550.2**

(22) Anmeldetag: **23.04.88**

(51) Int. Cl.⁴: **C07D 307/62**

---

(30) Priorität: **04.05.87 CH 1676/87**

(43) Veröffentlichungstag der Anmeldung:
**09.11.88 Patentblatt 88/45**

(84) Benannte Vertragsstaaten:
**BE CH DE FR GB IT LI NL SE**

(71) Anmelder: **F. HOFFMANN-LA ROCHE & CO.**
**Aktiengesellschaft**

**CH-4002 Basel(CH)**

(72) Erfinder: **Pauling, Horst, Dr.**
**Ruchholzstrasse 39**
**CH-4103 Bottmingen(CH)**
Erfinder: **Wehrli, Christof**
**Rheinstrasse 40**
**CH-4127 Birsfelden(CH)**

(74) Vertreter: **Urech, Peter, Dr. et al**
**Grenzacherstrasse 124 Postfach 3255**
**CH-4002 Basel(CH)**

---

(54) **Verfahren zur Herstellung von Derivaten der L-Ascorbinsäure.**

(57) Ein Verfahren zur Herstellung von L-Ascorbinsäure-2-sulfat und Salzen davon besteht darin, eine L-Ascorbinsäure mit geschützter 5,6-Stellung mit einem Salz eines tert. Amins mit Pyroschwefelsäure oder rauchender Schwefelsäure zu behandeln, die Schutzgruppe anschliessend abzuspalten und gegebenenfalls das erhaltene L-Ascorbinsäure-2-sulfat in ein Salz überzuführen.

**EP 0 289 894 A1**

## Verfahren zur Herstellung von Derivaten der L-Ascorbinsäure

Die Erfindung betrifft ein Verfahren zur Herstellung von Derivaten der L-Ascorbinsäure und zwar von L-Ascorbinsäure-2-sulfat und Salzen davon.

Das Verfahren ist dadurch gekennzeichnet, dass man eine L-Ascorbinsäure mit geschützter 5,6-Stellung mit einem Salz eines tert. Amins mit Pyroschwefelsäure oder rauchender Schwefelsäure behandelt und die Schutzgruppe anschliessend abspaltet, und gegebenenfalls das erhaltene L-Ascorbinsäure-2-sulfat in ein Salz überführt.

Geeigneterweise stellt man das Salz des tertiären Amins mit Pyroschwefelsäure oder rauchender Schwefelsäure in situ her, man verfährt also so, dass man die geschützte L-Ascorbinsäure mit einem Alkali-oder Erdalkalipyrosulfat in Anwesenheit des tertiären Amins umsetzt, bzw. dass man die geschützte L-Ascorbinsaure mit rauchender Schwefelsäure in Anwesenheit des tertiären Amins umsetzt. Als Alternative kann man jedoch das Salz im voraus herstellen, und zwar zweckmässigerweise durch Zugabe von rauchender Schwefelsäure zum Amin in einem Lösungsmittel, wie Methylenchlorid.

Als Schutzgruppen für die 5,6-Stellung der L-Ascorbinsäure fungieren zweckmässigerweise:

Ketale von niederen aliphatischen oder cycloaliphatischen Ketonen, wie beispielsweise von Aceton, Diäthylketon, Aethylmethylketon oder Cyclohexanon, Acetale von Aldehyden, wie beispielsweise Benzaldehyd oder Propionaldehyd.

Die bevorzugte L-Ascorbinsäure mit geschützter 5,6-Stellung ist die 5,6-Isopropyliden-L-ascorbinsäure.

Geeignete tertiäre Amine sind Trialkylamine wie Tri-nieder-alkylamine, z.B. Trimethyl-, Triäthyl-, Tri-n-Propyl-und Tri-isopropyl-amin; oder aromatische Amine, z.B. Pyridin und Picolin.

Als Alkalisalze der Pyroschwefelsäure fungieren die üblichen diesbezüglichen Salze, also das Lithium-, Natrium-, Kalium-oder Cäsiumsalz, als Erdalkalimetallsalz das Magnesium-, Calcium-, Strontium-oder Bariumsalz.

Der Gehalt an freiem SO₃ der rauchenden Schwefelsäure (Oleum) kann in einem weiten Bereich variieren, also z.B. 2-80 Gew.-%. insbesondere ca. 55-70 Gew.-% betragen. Pro Mol der geschützten Ascorbinsäure wird ein Mol freies SO₃ benötigt.

Das erfindungsgemässe Verfahren wird zweckmässigerweise in einem wasserfreien aprotischen, basischen Lösungsmittel durchgeführt. Geeignete solche Lösungsmittel sind z.B. Dimethylformamid, bzw. das oben erwähnte tertiäre Amin.

Das molare Verhältnis tertiäres Amin: Pyroschwefelsäure bzw. rauchende Schwefelsäure kann in einem breiten Bereich variieren, also z.B. ca. 1-10:1 betragen. In einer geeigneten Ausführungsform fungiert das tertiäre Amin als Lösungsmittel.

Man arbeitet zweckmässigerweise bei Raumtemperatur oder leicht erhöhter Temperatur, also bei Temperaturen von ca. 20-ca. 50°C.

Die Aufarbeitung des Primären Reaktionsproduktes, nämlich des L-Ascorbinsäure-2-sulfats mit geschützter 5,6-Stellung, umfasst zweckmässigerweise folgende Schritte: Entfernen von anorganischen Sulfaten, z.B. durch Fällung mit Calciumhydroxid, Behandlung mit einem stark sauren Ionentauscher zwecks Abspaltung der Schutzgruppe, und schliesslich Behandlung mit einer anorganischen Base, nämlich einem Alkalimetalloder Erdalkalimetallhydroxid zwecks Herstellung des im Vordergrund des Interesses stehenden Alkalimetall-bzw. Erdalkali-sulfats.

Aus der US-Patentschrift Nr. 3,954,809 ist ein Verfahren zur Sulfatierung von L-Ascorbinsäure zum entsprechenden 2-Sulfat unter Verwendung eines präformierten Amin-Schwefeltrioxid-Komplexes als Sulfatierungsmittel bekannt geworden. Nun ist die Herstellung dieses Amin-Schwefeltrioxid-Komplexes mit recht grossen Unannehmlichkeiten behaftet, da dabei zwangsläufig mit freiem SO₃ gearbeitet werden muss. Diese Unannehmlichkeiten fallen im vorliegenden Fall weg: Währenddem Oleum in der Schwefelsäure-herstellung im geschlossenen Kreislauf anfällt, bedingt bei der Herstellung des Amin-Schwefeltrioxid-Komplexes die Handhabung von freiem Schwefeltrioxid überdurchschnittliche Sicherheitsvorkehrungen.

Das erfindungsgemässe Verfahren wird durch die folgenden Beispiele veranschaulicht:

Beispiel 1

In einem 3 l-Vierhalsrundkolben mit Rührer, Thermometer und Inertbegasung werden 216 g (1,00 Mol) 5,6-Isopropyl iden-L-ascorbinsäure, 600 ml Pyridin 356 g (1.40 Mol) Kaliumdisulfat (Kaliumpyrosulfat), 400 ml Glaskugeln (∅ 3 mm) vorgelegt und 16 Stunden bei Raumtemperatur gerührt. Die Glaskugeln werden abgetrennt und mit 500 ml Wasser nachgewaschen. Die Suspension wird abgenutscht. Das Filtrat wird in einem 3 l-Sulfierkolben mit Rührer und Thermometer auf 50°C erwärmt. Dazu wird eine Suspension von 104 g Calciumhydroxid (96% = 1,4 Mol) in 500 ml Wasser zugegeben. Nach 1 Stunde Rühren bei Raumtemperatur wird das ausgefallene Calciumsulfat abgenutscht uno mit 2 x je 250 ml Wasser nachgewaschen. Das Filtrat wird am Rollverdampfer im Wasserstrahlvakuum auf ca. 700 ml eingeengt. Die Produktlösung wird durch einen Ionentauscher, nämlich 1,5 kg Dowex 50W X8 16-40 Mesh filtriert. Der Ionenaustauscher wird mit 1.6 l Wasser nachgewaschen. Das Eluat wird aufgefangen und bei Raumtemperatur bis zur vollständigen Hydrolyse der Isopropyliden-Schutzgruppe stehen gelassen. Die Lösung wird mit 184 ml 10N Kaliumhydroxid auf PH 8.0 ± 0.5 eingestellt und am Rollverdampfer im Wasserstrahlvakuum auf ca. 750 ml eingeengt. In einem 2 l-Rundkolben werden unter Rühren 850 ml Methanol zu dieser Lösung zugetropft. Das ausgefallene Produkt wird genutscht und der Filterkuchen mit einem Gemisch von 400 ml Methanol/wasser (55:45 G/G) nachgewaschen. Der Filterkuchen wird im Wasserstrahlvakuum getrocknet. Man erhält: 273,7 g Dikalium-L-ascorbat-2-sulfat • 2 H$_2$O, Gehalt: 96% (gemäss HPLC).

Beispiel 2

In einem 3 l-Vierhalsrundkolben mit Rührer und Thermometer werden unter Stickstoffatmosphäre 216 g (1,00 Mol) 5,6-Isopropylidenascorbinsäure, 600 ml Pyridin und 280 g (≙ 1,10 Mol freies SO$_3$) Pyridinpolysulfat vorgelegt und 18 Stunden bei Raumtemperatur gerührt. [Das "Pyridinpolysulfat" wird durch Zugabe von 100 ml rauchender Schwefelsäure (Oleum 60%) zu 240 ml Pyridin (Py) in 1 l Methylenchlorid hergestellt. Das resultierende "Pyridinpolysulfat" (378,4 g) hat die Zusammensetzung Py$_2$H$_2$S$_2$O$_7$•0,84 PySO$_3$.] Zum fast vollständig gelösten Gemisch werden 600 ml Wasser gegeben. Die Lösung wird auf 50°C erwärmt. Dazu werden eine Suspension von 136 g (96% = 1,84 Mol) Calciumhydroxid und 500 ml Wasser innert 30 Minuten zugegeben. Die Suspension wird 1 Stunde bei Raumtempertur gerührt und das ausgefallene Calciumsulfat genutscht und scharf abgepresst. Der Filterkuchen wird mit 200 ml Wasser angeteigt und nachgewaschen. Das Filtrat wird am Wasserstrahlvakuum auf ca. 500 ml eingeengt. Die Suspension wird genutscht und der Rückstand mit 300 ml Wasser nachgewaschen. Das Filtrat wird durch einen stark sauren Ionenaustauscher, nämlich 1,58 kg Dowex 50W X8, filtriert. Es wird mit 1,6 l Wasser nachgewaschen und das Eluat aufgefangen und bei Raumtemperatur bis zur vollständigen Hydrolyse der Isopropylidenschutzgruppe stehen gelassen. Die stark saure Lösung wird mit 214 ml 10N KOH auf pH 8,0±0,5 eingestellt und im Wasserstrahlvakuum auf ca. 750 ml eingeengt. Zur Produktlösung werden in einem 2 l-Rundkolben unter Rühren 850 ml Methanol zugetropft. Das ausgefallene Produkt wird genutscht und der Filterkuchen mit einem Gemisch von 400 ml Methanol/Wasser 55:45 G/G nachgewaschen. Das Produkt wird 18 Stunden im Wasserstrahlvakuum bei 45°C getrocknet. Man erhält 290,5 g Dikaliumascorbat-2-sulfat • 2 H$_2$O, Gehalt 92%.

**Ansprüche**

1. Verfahren zur Herstellung von L-Ascorbinsäure-2-sulfat und Salzen davon, dadurch gekennzeichnet, dass man eine L-Ascorbinsäure mit geschützter 5,6-Stellung mit einem Salz eines tert. Amins mit Pyroschwefelsäure oder rauchender Schwefelsäure behandelt und die Schutzgruppe anschliessend abspaltet, und gegebenenfalls das erhaltene L-Ascorbinsäure-2-sulfat in ein Salz überführt.

2. Verfahren nach Anspruch 1, dadurch gekennzeichnet, dass man die geschützte L-Ascorbinsäure mit einem Alkali-oder Erdalkalipyrosulfat in Anwesenheit des tertiären Amins umsetzt.

3. Verfahren nach Anspruch 1, dadurch gekennzeichnet, dass man die geschützte L-Ascorbinsäure mit rauchender Schwefelsäure in Anwesenheit des tertiären Amins umsetzt.

4. Verfahren nach einem der Ansprüche 1 bis 3, dadurch gekennzeichnet, dass ein Acetal oder Ketal als Schutzgruppe fungiert.

5. Verfahren nach Anspruch 4, dadurch gekennzeichnet, dass man 5,6-Isopropyliden-L-ascorbinsäure als Ausgangsmaterial verwendet.

6. Verfahren nach einem der Ansprüche 1 bis 5, dadurch gekennzeichnet, dass ein Trialkylamin oder Pyridin als tertiäres Amin benützt wird.

7. Verfahren nach einem der Ansprüche 1 bis 6, dadurch gekennzeichnet, dass man die L-Ascorbinsäure mit einem Alkalipyrosulfat, insbesondere mit K$_2$S$_2$O$_7$, behandelt.

8. Verfahren nach einem der Ansprüche 1 bis 7, dadurch gekennzeichnet, dass die rauchende Schwefelsäure ca. 2-80 Gew.-%, insbesondere ca. 55-70 Gew.-% freies SO$_3$ enthält.

9. Verfahren nach einem der Ansprüche 1 bis 8, dadurch gekennzeichnet, dass man in einem wasserfreien, aprotischen, basischen Lösungsmittel arbeitet.

10. Verfahren nach einem der Ansprüche 1 bis 8, dadurch gekennzeichnet, dass als Lösungsmittel das tertiäre Amin verwendet wird.

11. Verfahren nach einem der Ansprüche 1 bis 10, dadurch gekennzeichnet, dass das molare Verhältnis tertiäres Amin : Pyroschwefelsäure bzw. rauchende Schwefelsäure ca. 1-10:1 beträgt.

12. Verfahren nach einem der Ansprüche 1 bis 11, dadurch gekennzeichnet, dass man bei Raumtemperatur oder leicht erhöhter Temperatur arbeitet.

13. Verfahren nach einem der Ansprüche 1 bis 12, dadurch gekennzeichnet, dass das primäre Reaktionsprodukt nach Entfernen von anorganischem Sulfat durch Behandlung mit einem Ionentauscher und anschliessend mit einem Alkalimetall-oder Erdalkalimetallhydroxid in das Alkalimetall-oder Erdalkalimetall-L-ascorbinsäure-2-sulfat übergeführt wird.

Europäisches
Patentamt

**EUROPÄISCHER RECHERCHENBERICHT**

Nummer der Anmeldung

EP  88 10 6550

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACT, Band 79, Nr. 21, 26. November 1973, Seite 431, Zusammenfassung Nr. 126724p, Columbus, Ohio, US; S.F. QUADRI et al.: "Improved method of preparing L-ascorbic acid 2-sulfate", & CARBOHYD. RES. 1973, 29(1), 259-264 --- | 1,4-6 | C 07 D 307/62 |
| A | CHEMICAL ABSTRACT, Band 83, Nr. 3, 21. Juli 1975, Seite 543, Zusammenfassung 28523k, Columbus, Ohio, US; & US - H - 278 831 --- | 1,4-6 | |
| A | CHEMICAL ABSTRACT, Band 88, Nr. 3, 16. Januar 1978, Seite 674, Zusammenfassung 23294e, Columbus, Ohio, US; M. TSUJIMURA et al.:"Synthesis of ascorbic acid 2-sulfate and physicochemical properties", & JOSHI EIYO DAIGAKU KIYO 1975, 6, 35-44 --- | 1,4-6 | |
| D,A | US-A-3 954 809  (C.W. DEYOE et al.) * Ansprüche * --- | 1,6,13 | **RECHERCHIERTE SACHGEBIETE (Int. Cl.4)** C 07 D 307/00 |
| A | US-A-4 071 534  (E. HAYASHI) * Ansprüche * --- | 1,6 | |
| A | PATENT ABSTRACTS OF JAPAN, Band 1, Nr. 10, (C-76), 18. März 1977; & JP - A - 51 125 756 (KUMIAI CHEMICAL INDUSTRY K.K ) 02.11.1976 --- | 1,6 | |
| A | CHEMICAL ABSTRACTS, Band 85, Nr. 13, 27. September 1976, Seite 666, Zusammenfassung 94653v, Columbus, Ohio, US; & JP - A - 76 11757 ----- | 1,4 | |

Der vorliegende Recherchenbericht wurde für alle Patentansprüche erstellt

| Recherchenort | Abschlußdatum der Recherche | Prüfer |
|---|---|---|
| BERLIN | 22-07-1988 | HASS C V F |